# EUROPÄISCHE PATENTSCHRIFT

(11) **EP 1 032 445 B1**
(45) Veröffentlichungstag und Bekanntmachung des Hinweises auf die Patenterteilung: **15.09.2004**
(21) Anmeldenummer: 97913073.9
(22) Anmeldetag: 27.11.1997
(51) Int. Cl.: A61M 5/46, A61M 5/32

(54) **VERFAHREN UND VORRICHTUNG ZUR STEUERUNG DER EINDRINGTIEFE EINER INJEKTIONSNADEL**
METHOD AND DEVICE FOR CONTROLLING THE INTRODUCTION DEPTH OF AN INJECTION NEEDLE
PROCEDE ET DISPOSITIF DE COMMANDE DE LA PROFONDEUR DE PENETRATION D'UNE AIGUILLE POUR INJECTION

(43) Veröffentlichungstag der Anmeldung: 06.09.2000
(73) Patentinhaber: TecPharma Licensing AG, 3401 Burgdorf (CH)
(72) Erfinder: WICH, Horst, Cathedral City, CA 92234 (US)
(74) Vertreter: Schwabe - Sandmair - Marx
(86) Internationale Anmeldenummer: PCT/CH1997/000448
(87) Internationale Veröffentlichungsnummer: WO 1999/027986

(56) Entgegenhaltungen:
- AU-A- 5 861 273
- DE-A- 1 491 841
- DE-B- 1 022 758
- FR-A- 2 700 960
- US-A- 3 605 744
- US-A- 5 209 739

## Beschreibung

Die Erfindung betrifft ein Verfahren zur Einstellung der Eindringtiefe einer Injektionsnadel gemäss des Anspruchs 1 und eine Vorrichtung zur Durchführung dieses Verfahrens nach Anspruch 5.

Bei konventionellen Injektionsvorrichtungen besteht häufig das Problem, dass der Bediener - die Krankenschwester oder der Patient - die Einstechtiefe der Nadel nicht selbst steuern kann. Bei einem schlanken Kinde genügt im Gegensatz zu einem korpulenten Erwachsenen eine wesentlich kürzere Einstechtiefe. Wird die Injektionsnadel unnötigerweise tief in die Gewebehaut eingestochen, so resultieren vermeidbare Schmerzen daraus. Gerade bei Patienten, welche zur Medikamentenaufnahme auf häufige Injektionen angewiesen sind, z.B. Diabetiker führt dies zu einer Abneigung oder gar Angst vor der nächsten Injektion.

Beispielsweise ist aus der US-A-3 605 744 eine Injektionsvorrichtung gemäss dem Oberbegriff des Anspruch 2 bekannt, welche allerdings nur eine manuelle Injektion zulässt und somit wenig zuverlässig ist.

Die Nadel in DE-A-1 491 841 Figur 15 ist von einem hohlzylindrischen Distanzstück umgeben.

Der vorliegenden Erfindung liegt daher die Aufgabe zugrunde, ein Verfahren und eine Vorrichtung zur kontrollierten Steuerung der Eindringtiefe einer Injektionsnadel anzugeben, durch die es möglich ist, dass der Anwender die für die jeweilige Hautbeschaffenheit optimale Einstechtiefe einstellen kann.

Die Erfindung löst die gestellte Aufgabe mit einem Verfahren, gemäß Anspruchs 1 und einer Vorrichtung, welche die Merkmale des kennzeichnenden Teils des unabhängigen Anspruchs 5 aufweist.

Dank der Verkürzung des maximal möglichen Hubs H der Injektionsnadel durch ein Distanzstück, ist es möglich die Einstechtiefe einzustellen. Dies kann durch eine Reihe von Distanzstücken unterschiedlicher axialer Länge x erfolgen. Das konzentrisch um die Injektionsnadel angeordnete, hohlzylindrisches Distanzstück lässt sich auf einfache Art und Weise in die benutzte Nadelpenetrationsvorrichtung einschnappen, oder einklicken.

Die. Nadelpenetrationsvorrichtung ist entweder als separate, an eine konventionelle Injektionsvorrichtung koppelbare Einheit ausgebildet oder kann in die Injektionsvorrichtung selbst integriert sein.

Wenn ein Distanzstück in die Wegstrecke der Injektionsnadel eingeklickt oder eingeschraubt ist, so wird der maximale Hub H um den Betrag x verkürzt und es resultiert eine kürzere Einstechtiefe.

Bei einer bevorzugten Ausführungsform der Erfindung ist die Nadelpenetrationsvorrichtung eine separate an eine konventionelle Injektionsvorrichtung koppelbare Einheit welche einen hinteren longitudinalen Teil mit einer Durchstechnadel (welcher mit der Injektionsvorrichtung koppelbar ist) und einen vorderen longitudinalen Teil mit einer Injektionsnadel zur Applikation der flüssigen Substanz in die Gewebehaut umfasst. Zwischen der Durchstechnadel und der.Injektionsnadel ist eine flexible Verbindung vorgesehen.

Die Nadelpenetrationsvorrichtung ist über die Durchstechnadel mit der Penkarpule der Injektionsvorrichtung verbindbar, so dass über die Injektionsnadel die zu applizierende flüssige Substanz in die Gewebehaut injizierbar ist und der Vorgang des Einführens.der Injektionsnadel in die Gewebehaut nach manuellem Auslösen eines im hinteren, longitudinalen Teil untergebrachten Bedienelements der Nadelpenetrationsvorrichtung selbsttätig erfolgt und über die Injektionsvorrichtung die flüssige Substanz verabreicht werden kann.

Als Bedienelement dient eine Auslösehülse, die auf einem Durchstechnadelhalter, der die Durchstechnadel hält, längsverschiebbar angeordnet ist und mit einem in einer Anschlaghülse ebenfalls längsverschiebbaren Injektionsnadelhalter, der die Injektionsnadel hält, zusammenwirkt.

Das Einführen der Injektionsnadel in die Gewebehaut nach manuellem Auslösen der Auslösehülse erfolgt dadurch, dass der Injektionsnadelhalter zusammen mit der Injektionsnadel mittels der Kraft einer Feder selbsttätig in Richtung der Gewebehaut bewegt wird. Nach selbsttätigem Einführen der Injektionsnadel in die Gewebehaut kann sich der Bediener mittels der konventionellen Injektionsvorrichtung die zu verabreichende Dosis der flüssigen Substanz zuführen.

Die erfindungsgemässe Vorrichtung hat den wesentlichen Vorteil, dass sie eine grosse Bediensicherheit hinsichtlich der Eindringtiefe der Injektionsnadel in die Gewebehaut bietet.

Weitere vorteilhafte Ausgestaltungen der Erfindung ergeben sich aus den abhängigen Ansprüchen.

Ein Ausführungsbeispiel der Erfindung ist in den Figuren dargestellt.

Es zeigen:
Fig. 1: einen Längsschnitt durch eine erfindungsgemässe Vorrichtung vor dem Einsetzen des Distanzstückes, wobei sich die Nadelpenetrationsvorrichtung in der Ausgangsposition oder Ruhelage befindet;
Fig. 2: einen Längsschnitt durch die Vorrichtung gemäss Fig. 1 nach Einsetzen des Distanzstückes;
Fig. 3: einen Querschnitt längs der Linie III-III in Fig. 2;
Fig. 4: einen Längsschnitt durch die Vorrichtung gemäss Fig. 2 im entsicherten Zustand nach Abziehen der Sicherungskappe;
Fig. 5: einen Längsschnitt durch die Vorrichtung gemäss Fig. 2 nach manuellem Auslösen der Auslösehülse;
Fig. 6: einen Längsschnitt durch die Vorrichtung gemäss Fig. 2 (mit Distanzstück) nach Betätigung des Hubes der Nadelpenetrationsvorrichtung;
Fig. 7: einen Längsschnitt durch die Vorrichtung gemäss Fig. 1 (ohne Distanzstück) nach Betätigung des Hubes der Nadelpenetrationsvorrichtung;

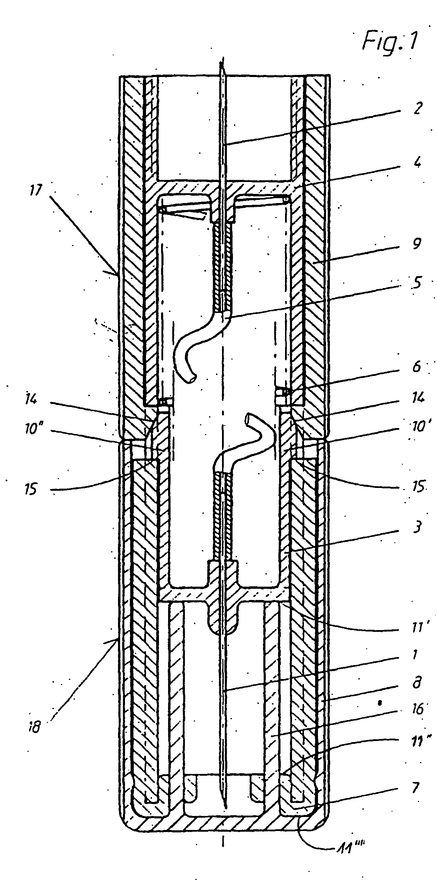

Fig. 1 zeigt eine Nadelpenetrationsvorrichtung 17, 18 zum Injizieren einer flüssigen Substanz. Die Nadelpenetrationsvorrichtung 17,18 besteht im wesentlichen aus einem hinteren longitudinalen Teil 17 mit einer Durchstechnadel 2 und einem vorderen longitudinalen Teil 18 mit einer Injektionsnadel 1 zur Applikation der flüssigen Substanz in die Gewebehaut sowie einer flexible Verbindung 5 zwischen der Durchstechnadel 2 und der Injektionsnadel 1.

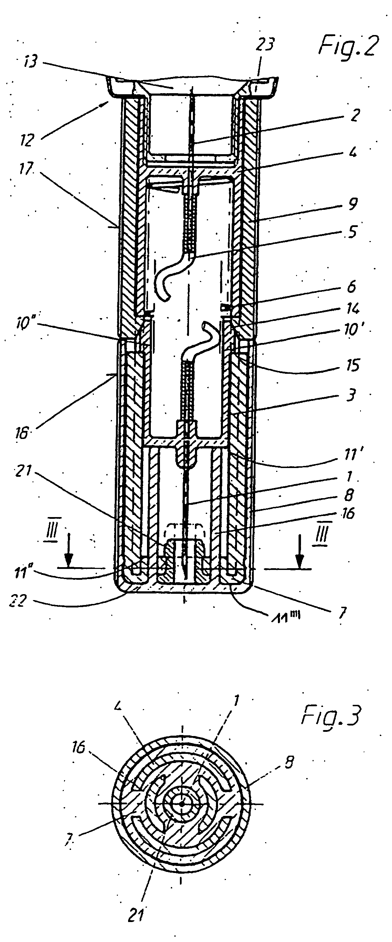

Die Nadelpenetrationsvorrichtung 17,18 ist mit der konventionellen Injektionsvorrichtung 12 koppelbar, beispielsweise durch einen Gewindeverschluss oder durch eine Rast-Nocken-Aufsteckverbindung 23 (Fig. 2). Über die am einen Ende der Nadelpenetrationsvorrichtung 17,18 angebrachte Dürchstechnädel 2 ist die Nadelpenetrationsvorrichtung 17,18 mit der Penkarpule 13. der konventionellen Injektionsvorrichtung 12 in Verbindung. Über die am anderen Ende der Nadelpenetrationsvorrichtung 17,18 angebrachte Injektionsnadel 1 ist die zu applizierende flüssige Substanz in die Gewebehaut injizierbar. Zwischen Durchstechnadel 2 und Injektionsnadel 1 ist die flexible Verbindung 5 für den Transport der flüssigen Substanz vorgesehen. Der Vorgang des Einführens der Injektionsnadel 1 in die Gewebehaut erfolgt nach manuellem Auslösen eines Bedienelements in Form der Auslösehülse 9 der Nadelpenetrationsvorrichtung 17,18 selbsttätig, und anschliessend kann über die konventionelle Injektionsvorrichtung 12 die flüssige Substanz verabreicht werden. Die Auslösehülse 9 ist auf dem Durchstechnadelhalter 4, der die Durchstechnadel 2 hält, längsverschiebbar angeordnet und wirkt mit dem im Durchstechnadelhalter 4 sowie in der Anschlaghülse 7 ebenfalls längsverschiebbaren Injektionsnadelhalter 3, der die Injektionsnadel 1 hält, zusammen. Das Einführen der Injektionsnadel 1 in die Gewebehaut erfolgt nach manuellem Auslösen der Auslösehülse 9 mittels der Kraft der Feder 6, indem der Injektionsnadelhalter 3 mit der Injektionsnadel 1 selbsttätig gegen die Gewebehaut bewegt wird. Dabei stützt sich die Feder 6 mit ihrem einen Ende am ortsfesten Durchstechnadelhalter 4 ab und bewegt mit ihrem anderen Ende den Injektionsnadelhalter 3 mit der Injektionsnadel 1 in Richtung der Gewebehaut, wodurch in diese die Injektionsnadel 1 selbsttätig eingeführt wird. Das Zusammenwirken der Auslösehülse 9 und dem Injektionsnadelhalter 3 beim manuellen Auslösen der Auslösehülse 9 erfolgt über die konischen Abschrägungen 14, durch die der Injektionsnadelhalter 3 im Durchmesser zusammengedrückt wird und so die Verriegelung, bestehend aus dem Anschlag 15 der Anschlaghülse 7 und den Haltenocken 10', 10'' des Injektionsnadelhaltes 3 aufgehoben wird und dass somit die Feder 6 ihre Schiebewirkung entfalten kann. Der Injektionsnadelhalter 3 wird mit der Injektionsnadel 1 nach dem manuellen Auslösen der Auslösehülse 9 durch die Kraft der Feder 6 solange in Richtung der Gewebehaut selbsttätig bewegt, bis die Stirnfläche 11' des Injekcionsnadelhalters 3 an der Stirnfläche 11" der Anschlaghülse 7 zum Anliegen kommt. Nach erfolgter Injizierung ist der Injektionsnadelhalter 3 mit der Injektionsnadel 1 mittels der die Anschlaghülse 7 umgebenden Sicherungskappe 8 manuell gegen die Kraft der Feder 6 zurückschiebbar, bis die Verriegelung, bestehend aus dem Anschlag 15 der Anschlaghülse 7 und den Haltenocken 10', 10'' des Injektionsnadelhalter 3 wieder wirksam ist. Gleichzeitig wird die Auslösehülse 9 mittels der konischen Abschrägungen 14 in Richtung der konventionellen Injektionsvorrichtung 12 durch die Sicherungskappe 8 längsverschoben, so dass die Auslösehülse 9 in die Ausgangsposition oder Ruhelage zurückbewegt wird und für eine erneute manuelle Auslösung bereit ist. Zum Zurückschieben des Injektionsnadelhalters 3 mit der Injektionsnadel 1 mittels der Sicherungskappe 8, ist letztere in ihrem Innern mit einem die Injektionsnadel 1 umgebenden Schutzzylinder 16 versehen, der zum einen die Injektionsnadel 1 schützt. Zum anderen hat der Schutzzylinder 16 die Funktion, dass er mit seinem offenen Ende an der Stirnfläche 11' des Injektionsnadelhalters 3 anliegt und somit beim Aufschieben der Sicherungskappe 8 in die Injektionsnadel 1 schützendem Zustand den Injektionsnadelhalter 3 samt Injektionsnadel sowie die Auslösehülse 9 in die Ausgangsposition zurückschiebt.

Die Funktionsweise der erfindungsgemässen Nadelpenetrationsvorrichtung 17,18 ist folgende:

Die Nadelpenetrationsvorrichtung 17,18 wird mit der konventionellen Injektionsvorrichtung 12 mechanisch gekoppelt durch z.B. einen Gewindeverschluss oder durch eine Rast-Nocken-Auf steckverbindung oder ähnlichem. Dabei durchsticht die Durchstechnadel 2 die Membran der Penkarpule und sorgt somit dafür, dass die zu injizierende flüssige Substanz in die Nadelpenetrationsvorrichtung fliessen kann. Der Bediener führe nun letztere an eine bevorzugte Gewebehautstelle, löst anschliessend die Auslösehülse 9 aus, indem er diese in Richtung Gewebehaut. nach unten längsverschiebt, bis die Verriegelung, bestehend aus dem Anschlag 15 der Anschlaghülse 7 und den Haltenocken 10', 10'' des Injektionsnadelhalters 3 aufgehoben wird. Dadurch kann die Feder 6 ihre Kraft entfalten und bewegt den Injektionsnadelhalter 3 mit der Injektionsnadel 1 solange in Richtung der Gewebehaut selbsttätig, bis die Stirnfläche 11' des Injektionsnadelhalters 3 an der Stirnfläche 11'' der Anschlaghülse 7 zum Anliegen kommt. Dadurch ist die Injektionsnadel 1 selbsttätig in die Gewebehaut eingeführt worden. Jetzt kann der Bediener sich mittels der konventionellen Injektionsvorrichtung 12 die zu verabreichende Dosis an flüssiger Substanz applizieren. Nach erfolgter Injizierung ist der Injektionsnadelhalter 3 mit der Injektionsnadel 1 mittels Sicherungskappe 8 manuell gegen die Kraft der Feder 6 zurückschiebbar. Die Verriegelung wird wieder wirksam und gleichzeitig kehrt die Auslasshülse 9 in ihre Ausgangsposition oder Ruhelage zurück.

Der Durchstechnadelhalter 3 kann im Bereich der konischen Abschrägungen 14 vorzugsweise geschlitzt sein zur Elastizitätsverbesserung, wenn er.beim Zusammenwirken mit der Auslösehülse 9 beim Auslösevorgang zusammengedrückt wird, um die Verriegelung aufzuheben.

Die Nadelpenetrationsvorrichtung 17, 18 besitzt an der Anschlaghülse 7, wie in den Fig. 1 bis 7 zu erkennen ist, eine der Injektionsvorrichtung 12 diametral gegenüberliegende stirnseitige Endfläche 11"".

Die Fig. 2 und 3 zeigen die Nadelpenetrationsvorrichtüng 17,18, in welche ein hohlzylindrisches Distanzstück 21 konzentrisch um die Injektionsnadel 1 herum in den vorderen longitudinalen Teil 18 eingeklickt und dort befestigt ist. Zu diesem Zweck weist dass Distanzstück 21 eine periphere Hinterschneidung 22 auf. Das hohlzylindrische Distanzstück 21 besitzt eine äußere, endseitige Stirnfläche 21a und eine, dieser diametral axial gegenüberliegende innere Stirnfläche 11"'. Mit der äußeren Stirnfläche 21a schließt das Distanzstück 21 mit der stirnseitigen Endfläche 11 "." . der Nadelpenetrationsvorrichtung 17, 18 bzw. der Anschlaghülse 7 ab. Mit der inneren Stirnfläche 11 ' ' ' steht das hohlzylindrische Distanzstück 21 über eine Stirnfläche 11 "der Anschlaghülse 7 über und erstreckt sich um einen Betrag x koaxial um die Injektionsnadel 1 in die Anschlaghülse 7 hinein. Das einmal eingebrachte Distanzstück 21, welches vorzugsweise aus Kunststoff gefertigt ist, verbleibt in der als Einweginstrument vorgesehenen Nadelpenetrationsvorrichtung 17,18 und wird somit zusammen mit dieser entsorgt.

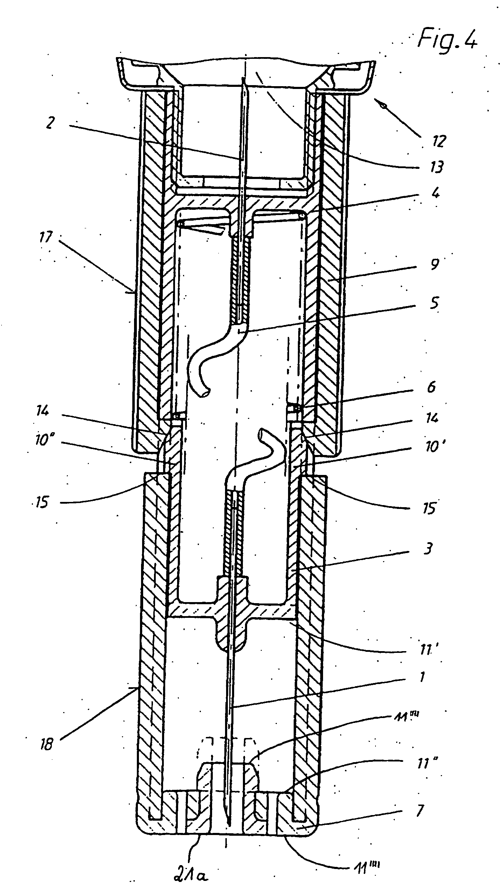

Die. Fig. 4 zeigt einen Längsschnitt der Nadelpenetrationsvorrichtung 17,18 im entsicherten Zustand nach Abziehen der Sicherungskappe 8. Bis auf diese Sicherungskappe 8 ist Fig. 4 im wesentlichen identisch mit Fig. 2. Die Bezugszeichen sind in beiden Figuren identisch.

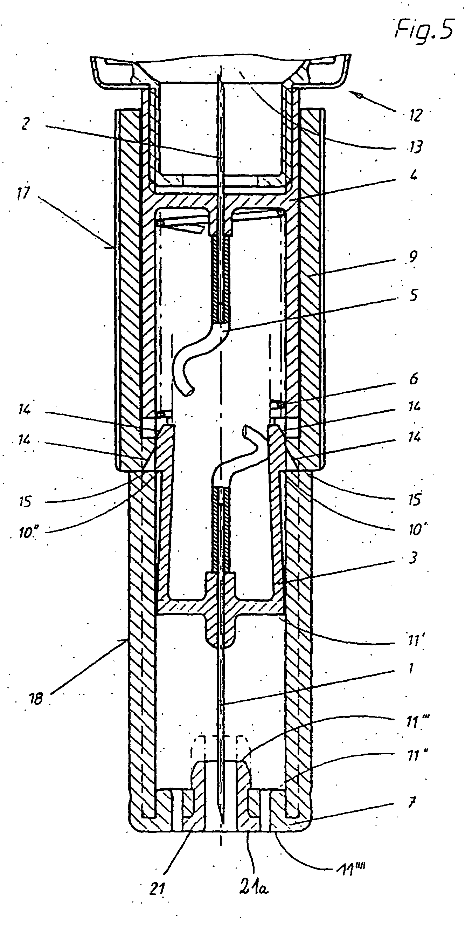

Fig. 5 zeigt einen Längsschnitt der Nadelpeneträtionsvorrichtung 17,18 nach manuellem Auslösen der Auslösehülse 9. Diese Figur zeigt, wie unmittelbar nach Aufheben der verriegelung, bestehend aus dem Anschlag 15 der Anschlaghülse 7 und den Haltenocken 10', 10'' des Injektionsnadelhalters 3, durch das Zusammenwirken der konischen Abschrägungen 14 von Injektions- nadelhalter 3 und Auslösehülse 9 der Injektionsnadelhalter 3 federnd zusammengedrückt wird, damit die Feder 6 ihre Kraft entfalten kann, und der selbsttätige Einführvorging der Injektionsnadel 1 in die Gewebehaut, beginnen kann.

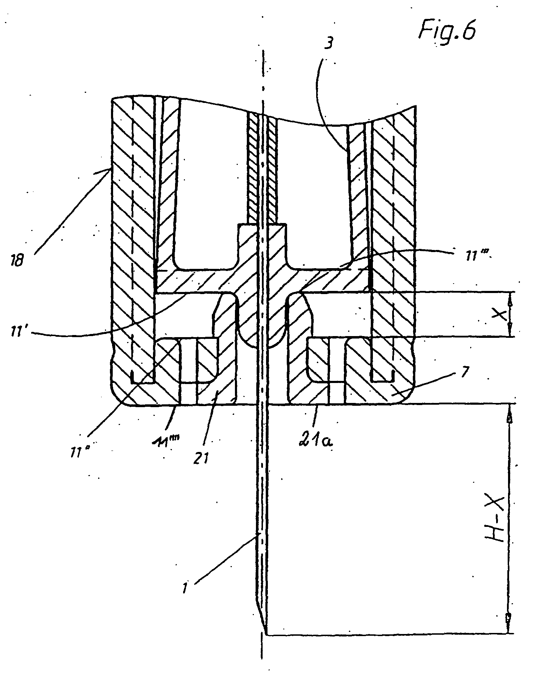

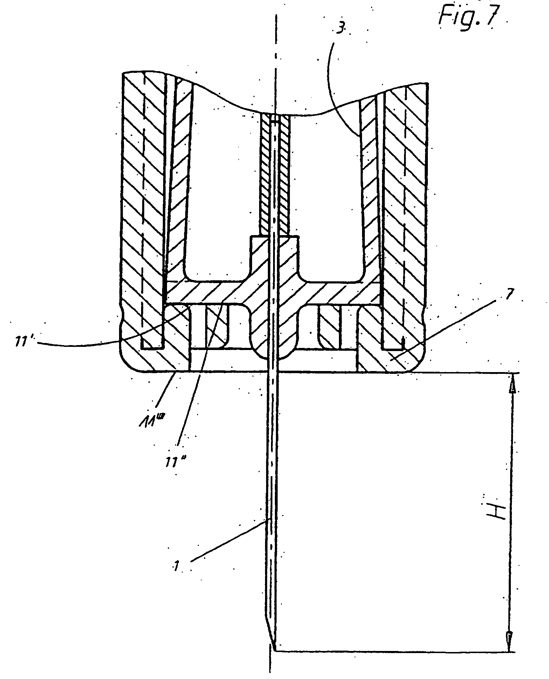

Im Gegensatz zur Nadelpenetrationsvorrichtung ohne eingesetztes Distanzstück 21, kann hier der Injektionsnadelhalter 3 nur bis zur .Stirnfläche 11''' vorgeschoben werden. Die Einstichtiefe wird damit unterschiedlich verkürzt. Wie in den Fig. 6/7 dargestellt stösst die Stirnfläche 11' des Injektionsnadelhalters 3 durch das Einsetzen des Distanzstücks 21 (Fig. 6) bereits früher an die Stirnfläche 11''. der Anschlaghülse 7 und zwar um den Abstand x zwischen Stirnfläche 11"' und Stirnfläche 11". Bei eingesetztem Distanzstück 21 (Fig. 6) beträgt somit die Einstichtiefe H-x gegenüber der in Fig. 7 gezeigten Situation ohne Distanzstück mit einer Einstichtiefe H.

Die erfindungsgemässe Vorrichtung ist anwendbar zum Verabreichen von vorzugsweise Medikamenten. Sie kann darüberhinaus überall dort angewendet werden, wo eine flüssige Substanz in einen Körper einzubringen ist und die Injektionsnadel selbsttätig und mit kontrollierter Steuerung der Eindringtiefe der Injektionsnadel eingeführt werden soll.

## Patentansprüche

1. Verfahren zur Einstellung der Eindringtiefe einer Injektionsnadel (1), welche innerhalb einer Nadelpenetrationsvorrichtung (17, 18), gegenüber einer damit gekoppelten oder diese umfassende Injektionsvorrichtung (12), axial beweglich angeordnet ist, wobei der maximale Hub der Injektionsnadel (1) den Wert H beträgt,
wobei der maximal mögliche Hub H der Injektionsnadel (1) durch ein konzentrisch um die Injektionsnadel (1) befestigtes und axial in die Nadelpenetrationsvorrichtung (17, 18) vorstehendes, hohlzylindrisches Distanzstück (21) verkürzt wird, welches mit einer Stirnfläche um eine axiale Länge x in die Nadelpenetrationsvorrichtung über eine innere Stirnfläche der Nadelpenetration Vorrichtung vorsteht und dadurch der Hub H der Injektionsnadel (1) auf den Wert H-x verkürzt wird.

2. Verfahren nach Anspruch 1, **dadurch gekennzeichnet, dass** ein außenseitig mit der Nadelpenetrationsvorrichtung (17, 18) abschließendes hohlzylindrisches Distanzstück (21) verwendet wird.

3. Verfahren nach Anspruch 1 oder 2, **dadurch gekennzeichnet, dass** das Distanzstück (21) mit einer äußeren endseitigen Stirnfläche (21a) im Bereich einer äußeren Stirnfläche (11 " ") der Nadelpenetrationsvorrichtung mit dieser Fläche (11"'') abschließend eingefügt und befestigt wird.

4. Verfahren nach einem der vorhergehenden Ansprüche, **dadurch gekennzeichnet, dass** das Zusammenwirken einer Auslösehülse (9) und eines Injektionsnadelhalters (3) beim manuellen Auslösen der Auslösehülse (9) über konische Abschrägungen (14) erfolgt, durch die der Injektionsnadelhalter (3) im Durchmessei zusammengedrückt wird und so eine lösbare Verriegelung, bestehend aus einem Anschlag (15) der Anschlaghülse (7) und Haltenocken (10', 10") des Injektionsnadelhalters (3) aufgehoben wird, sodass die Feder (6) ihre Schiebewirkung entfalten kann.

5. Vorrichtung, zur Einstellung der Eindringtiefe einer Injektionsnadel (1), und zur Durchführung des Verfahrens nach einem oder mehreren der Ansprüche 1 bis 4, welche innerhalb einer Nadelpenetrationsvorrichtung (17, 18 ) gegenüber einer damit gekoppelten oder diese umfassenden Injektionsvorrichtung (12) axial beweglich angeordnet ist, wobei der maximale Hub der Injektionsnadel (1) den Wert H beträgt, und wobei sie eine derartige Nadelpenetrationsvorrichtung (17, 18) mit einer axial beweglichen Injektionsnadel (1) umfasst, in welche ein hohlzylindrisches Distanzstück (21) konzentrisch um die Injektionsnadel (1) eingeführt und befestigt ist, **dadurch gekennzeichnet dass** Distanzstück (21) mit einer Stirnfläche (11"') über eine innere Stirnfläche (11") der Nadelpenetrationsvorrichtung (17, 18)nach innen in die Nadelpenetrationsvorrichtung (17, 18), den maximalen Hub H der Injektionsnadel (1) verkürzend, vorsteht.

6. Vorrichtung nach Anspruch 5, **dadurch gekennzeichnet, dass** die Nadelpenetrationsvorrichtung (17, 18) einen Schnapp- oder Klickverschluss aufweist, in welchen das Distanzstück (21) eingeschnappt oder eingeklickt ist.

7. Vorrichtung nach Anpruch 5, **dadurch gekennzeichnet, dass** die Nadelpenetrationsvorrichtung (17, 18) folgende Komponenten umfasst:
a) einen hinteren longitudinalen Teil (17) mit einer Durchstechnadel (2), welche mit einer Injektionsvorrichtung (12) koppelbar ist;
b) einen vorderen longitudinalen Teil (18) mit der Injektionsnadel (1) zur Applikation der flüssigen Substanz in die Gewebehaut; und
c) eine flexible Verbindung (5) zwischen der Durchstechnadel (2) und der Injektionsnadel (1).

8. Vorrichtung nach Anspruch 7, **dadurch gekennzeichnet, dass** im hinteren, longitudinalen Teil (7) der Nadelpenetrationsvorrichtung (17, 18). ein Bedienelement (9) der Nadelpenetrationsvorrichtung (17, 18) angeordnet ist, nach dessen manueller Auslösung die Injektionsnadel (1) in die Gewebehaut eingeführt wird.

9. Vorrichtung nach Anspruch 8, **dadurch gekennzeichnet, dass** das Bedienelement (9) eine Auslösehülse (9) ist, die auf einem Durchstechnadelhalter (4), der die Durchstechnadel (2) hält, längsverschiebbar angeordnet ist und mit einem in einer Anschlaghülse (7) ebenfalls längsverschiebbaren Injektionsnadelhalter (3), der die Injektionsnadel (1) hält, zusammenwirkt.

10. Vorrichtung nach Anspruch 9, **dadurch gekennzeichnet, dass** eine Feder (6) vorhanden ist, wobei sich die Feder (6) mit ihrem einen Ende am ortsfesten Durchstechnadelhalter (4) abstützt und mit ihrem anderen Ende am Injektionsnadelhalter (3) abstützt, sodass der Injektiönsnadelhalter (3) mit der Injektionsnadel (1) in Richtung der Gewebehaut bewegbar ist und in diese die Injektionsnadel (1) durch die Federkraft der Feder (6) einführbar ist.

11. Vorrichtung nach Anspruch 9, **dadurch gekennzeichnet, dass** eine lösbare Verriegelung, bestehend aus einem Anschlag (15) der Anschlaghülse (7) und Haltenocken (10', 10") des Injektionsnadelhalters (3) vorhanden ist, wobei die Auslösehülse (9) konische Abschrägungen (14) besitzt, wobei die Auslösehülse (9) und der Injektionsnadelhalter (3) beim manuellen Auslösen der Auslösehülse (9) über die konischen Abschrägungen (14) derart erfolgt, dass durch das Zusammendrücken des Injektionsnadelhalters (3) die lösbare Verriegelung, bestehend aus dem Anschlag (15) und dem Haltenocken (10', 10") aufgehoben wird, sodass die Feder (6) ihre Schiebewirkung entfalten kann.

12. Vorrichtung nach Anspruch 9, **dadurch gekennzeichnet, dass** eine Sicherungskappe (8) vorhanden ist, welche die Anschlaghülse (7) zum Zurückschieben des Injektionsnadelhalters (3) mit der Injektionsnadel (1) gegen die Kraft der Feder (6) vorhanden ist.

13. Vorrichtung nach einem der Ansprüche 5 bis 12, **dadurch gekennzeichnet, dass** die Ankopplung der Nädelpenetrationsvorrichtung (17, 18) an die Injektionsvorrichtung (12) mittels eines Gewindeverschlusses oder einer Rast-Nocken-Aufsteckverbindung erfolgt.

14. Vorrichtung nach Anspruch 12, **dadurch gekennzeichnet, dass** die Sicherungskappe (8) in ihrem Inneren einen die Injektionsnadel (1) umgebenden Schutzzylinder (16) aufweist.

15. Vorrichtung nach einem der Ansprüche 7 bis 14, **dadurch gekennzeichnet, dass** die Nadelpenetrationsvorrichtung (17, 18) eine endseitige, der Injektionsvorrichtung (12) gegenüberliegende Stirnfläche (11"") besitzt, und das Distanzstück (21) mit einer außenseitigen Stirnfläche (21a) mit der Stirnfläche (11"") abschließt.

## Claims

1. A method for setting the penetration depth of an injection needle (1) which is arranged within a needle penetration device (17, 18) such that it can be moved axially relative to an injection device (12) coupled to or comprising said needle penetration device (17, 18), wherein the maximum stroke of said injection needle (1) has the value H,
and wherein said maximum possible stroke H by the injection needle (1) is shortened by a hollow-cylindrical distancing piece (21) which is concentrically fixed around the injection needle (1) and axially protrudes into the needle penetration device (17, 18), and protrudes via a facing surface into the needle penetration device by an axial length x beyond an inner facing surface of the needle penetration device, which shortens the stroke H of the injection needle (1) to the value H-x.

2. The method as set forth in claim 1, **characterised in that** a hollow-cylindrical distancing piece (21) is used which terminates on the outer side with the needle penetration device (17, 18).

3. The method as set forth in claim 1 or 2, **characterised in that** an outer end facing surface (21a) of the distancing piece (21) is joined and fixed in the area of an outer facing surface (11"") of the needle penetration device and terminates with said surface (11"").

4. The method as set forth in any one of the preceding claims, **characterised in that** a triggering sleeve (9) and an injection needle holder (3) co-operate via conical chamfers (14) when said triggering sleeve (9) is manually triggered, which compresses said injection needle holder (3) in its diameter and thus deactivates a releasable latch consisting of a stopper (15) of the stopper sleeve (7) and holding cam (10', 10") of the injection needle holder (3), such that the spring (6) can deploy its pushing effect.

5. A device for setting the penetration depth of an injection needle (1) and for performing the method as set forth in one or more of claims 1 to 4, arranged within a needle penetration device (17, 18) such that it can be moved axially relative to an injection device (12) coupled to or comprising said needle penetration device (17, 18), wherein the maximum stroke of said injection needle (1) has the value H, and wherein said device comprises such a needle penetration device (17, 18) with an axially moving injection needle (1), into which a hollow-cylindrical distancing piece (21) is inserted and fixed concentrically around the injection needle (1), **characterised in that** said distancing piece (21) protrudes inwards via a facing surface (11'") into the needle penetration device (17, 18) via an inner facing surface (11'') of the needle penetration device (17, 18), shortening the maximum stroke H of the injection needle (1).

6. The device as set forth in claim 5, **characterised in that** the needle penetration device (17, 18) comprises a snap lock or click lock into which the distancing piece (21) is snapped or clicked.

7. The device as set forth in claim 5, **characterised in that** the needle penetration device (17, 18) comprises the following components:
a) a rear longitudinal portion (17) comprising a piercing needle (2) which can be coupled to an injection device (12);
b) a front longitudinal portion (18) comprising the injection needle (1) for administering the liquid substance into the tissue skin; and
c) a flexible connection (5) between said piercing needle (2) and the injection needle (1).

8. The device as set forth in claim 7, **characterised in that** an operating element (9) of the needle penetration device (17, 18) is arranged in said rear longitudinal portion (17) of the needle penetration device (17, 18), wherein the injection needle (1) is introduced into the tissue skin once said operating element (9) has been manually triggered.

9. The device as set forth in claim 8, **characterised in that** the operating element (9) is a triggering sleeve (9) which is arranged, such that it can be longitudinally shifted, on a piercing needle holder (4) which holds the piercing needle (2), and co-operates with an injection needle holder (3) which holds the injection needle (1) and can also be longitudinally shifted in a triggering sleeve (7).

10. The device as set forth in claim 9, **characterised in that** a spring (6) is provided, wherein said spring (6) is supported at one end on the positionally stable piercing needle holder (4) and at its other end on the injection needle holder (3), such that the injection needle holder (3) including the injection needle (1) can be moved towards the tissue skin and the injection needle (1) can be introduced into the tissue skin by the spring force of the spring (6).

11. The device as set forth in claim 9, **characterised in that** a releasable connection is provided, consisting of a stopper (15) of the stopper sleeve (7) and holding cam (10', 10") of the injection needle holder (3), wherein the triggering sleeve (9) comprises conical chamfers (14) and the triggering sleeve (9) and the injection needle holder (3) co-operate via said conical chamfers (14) when said triggering sleeve (9) is manually triggered, such that the releasable latch, consisting of said stopper (15) and holding cam (10', 10"), is deactivated by compressing the injection needle holder (3), such that the spring (6) can deploy its pushing effect.

12. The device as set forth in claim 9, **characterised in that** a safety cap (8) surrounding the stopper sleeve (7) is provided for retracting the injection needle holder (3) including the injection needle (1), against the force of the spring (6).

13. The device as set forth in any one of claims 5 to 12, **characterised in that** the needle penetration device (17, 18) is coupled to the injection device (12) by means of a threaded lock or a locking cam plugging connection.

14. The device as set forth in claim 12, **characterised in that** said safety cap (8) comprises, in its interior, a protective cylinder (16) surrounding the injection needle (1).

15. The device as set forth in any one of claims 7 to 14, **characterised in that** the needle penetration device (17, 18) comprises an end facing surface (11"") opposite the injection device (12), and **in that** the distancing piece (21) forms a seal with the facing surface (11"") via its facing surface (21a) on the outer side.

## Revendications

1. Procédé de réglage de la profondeur de pénétration d'une aiguille pour injection (1) qui est montée, à l'intérieur d'un dispositif de pénétration de l'aiguille (17, 18), de manière axialement mobile par rapport à un dispositif de injection (12) couplé au dispositif de pénétration de l'aiguille (17, 18) ou le comprenant, la course maximale de l'aiguille pour injection (1) ayant la valeur H,
la course maximale possible H de l'aiguille pour injection (1) étant raccourcie par une entretoise (21) cylindrique creuse, fixée concentriquement autour de l'aiguille pour injection (1) et dépassant axialement dans le dispositif de pénétration de l'aiguille (17, 18), cette entretoise dépassant, d'une longueur axiale x dans le dispositif de pénétration de l'aiguille au-dessus d'une face frontale intérieure du dispositif de pénétration de l'aiguille, et la course H de l'aiguille pour injection (1) étant raccourcie à la valeur H-x.

2. Procédé selon la revendication 1, **caractérisé en ce qu'**on utilise une entretoise (21) cylindrique creux se terminant à l'extérieur par le dispositif de pénétration de l'aiguille (17, 18).

3. Procédé selon la revendication 1 ou 2, **caractérisé en ce que** l'entretoise (21) est insérée de manière occlusive et fixé avec une face frontale externe terminale (21a) dans la région d'une face frontale extérieure (11"") du dispositif de pénétration de l'aiguille avec cette face (11"").

4. Procédé selon l'une quelconque des revendications précédentes, **caractérisé en ce que** l'interaction d'un manchon de déclenchement (9) et d'un porte-aiguille pour injection (3) lors du déclenchement manuel du manchon de déclenchement (9) intervient par l'intermédiaire de chanfreins coniques (14), qui compriment radialement le porte-aiguille pour injection (3) entraînant ainsi le soulèvement d'un verrou détachable, constitué d'une butée (15) du manchon de butée (7) et d'une came de retenue (10', 10") du porte-aiguille pour injection (3), de sorte que le ressort (6) peut déployer son action coulissante.

5. Dispositif de réglage de la profondeur de pénétration d'une aiguille pour injection (1) et permettant la réalisation du procédé selon l'une quelconque ou plusieurs des revendications 1 à 4, qui est montée, à l'intérieur d'un dispositif de pénétration de l'aiguille (17, 18), de manière axialement mobile par rapport à un dispositif de injection (12) couplé au dispositif de pénétration de l'aiguille (17, 18) ou le comprenant, la course maximale de l'aiguille pour injection (1) ayant la valeur H, et comportant un tel dispositif de pénétration de l'aiguille (17, 18) ayant une aiguille pour injection (1) mobile axialement, dans lequel une entretoise cylindrique creuse (21) est inséré concentriquement autour de l'aiguille pour injection (1) et fixé, **caractérisé en ce que** l'entretoise (21) dépasse avec une face frontale (11 "') au-dessus d'une face frontale intérieure (11") du dispositif de pénétration de l'aiguille (17, 18) vers l'intérieur du dispositif de pénétration de l'aiguille (17, 18) tout en raccourcissant la course maximale H de l'aiguille pour injection (1).

6. Dispositif selon la revendication 5, **caractérisé en ce que** le dispositif de pénétration de l'aiguille (17, 18) présente une fermeture à cliquet ou à crans, dans laquelle l'entretoise (21) vient s'encliqueter ou se clipser.

7. Dispositif selon la revendication 5, **caractérisé en ce que** le dispositif de pénétration de l'aiguille (17, 18) comporte les composants suivants :
a) une partie postérieure longitudinale (17) ayant une aiguille de percée (2), qui peut être couplée à un dispositif pour injection (12) ;
b) une partie antérieure longitudinale (18) ayant l'aiguille pour injection (1) permettant l'application de la substance liquide dans la peau du tissu ; et
c) un liaison flexible (5) entre l'aiguille de percée (2) et l'aiguille pour injection (1).

8. Dispositif selon la revendication 7, **caractérisé en ce que** dans la partie postérieure longitudinale (7) du dispositif de pénétration de l'aiguille (17, 18) est disposé un élément de manoeuvre (9) du dispositif de pénétration de l'aiguille (17, 18) après le déclenchement manuel duquel l'aiguille pour injection (1) est introduite dans la peau du tissu.

9. Dispositif selon la revendication 8, **caractérisé en ce que** l'élément de manoeuvre (9) est un manchon de déclenchement (9) qui est disposé sur un porte-aiguille de percée (4), qui tient l'aiguille de percée (2), de manière à coulisser longitudinalement et interagit avec un porte-aiguille pour injection (3), qui tient l'aiguille pour injection (1), également coulissante dans le sens de la longueur dans un manchon de butée (7).

10. Dispositif selon la revendication 9, **caractérisé en ce qu'**il comporte un ressort (6), le ressort (6) s'appuyant, avec une de ses extrémités sur le porte-aiguille de percée (4) stationnaire et, avec son autre extrémité, s'appuyant sur le porte-aiguille pour injection (3), de sorte que le porte-aiguille pour injection (3) puisse être déplacé avec l'aiguille pour injection (1) dans le sens de la peau du tissu et l'aiguille pour injection (1) puisse être introduite dans celle-ci par la force élastique du ressort (6).

11. Dispositif selon la revendication 9, **caractérisé en ce qu'**il comporte un verrou détachable, constitué d'une butée (15) du manchon de butée (7) et de cames de retenue (10', 10") du porte-aiguille pour injection (3), le manchon de déclenchement (9) possédant des chanfreins coniques (14), le manchon de déclenchement (9) et le porte-aiguille pour injection (3) intervenant lors du déclenchement manuel du manchon de déclenchement (9) par l'intermédiaire des chanfreins coniques (14) de manière à ce que la compression du porte-aiguille pour injection (3) permette au verrou détachable, constitué de la butée (15) et de la came de retenue (10', 10") de se soulever de manière à ce que le ressort (6) puisse déployer son action coulissante.

12. Dispositif selon la revendication 9, **caractérisé en ce qu'**il comprend un chapeau de sûreté (8), qui est monté sur le manchon de butée (7) pour reculer le porte-aiguille pour injection (3) avec l'aiguille pour injection (1) à l'encontre de la force du ressort (6).

13. Dispositif selon l'une quelconque des revendications 5 à 12, **caractérisé en ce que** le couplage du dispositif de pénétration de l'aiguille (17, 18) au dispositif pour injection (12) est assuré au moyen d'une fermeture filetée ou d'une liaison emboîtable à came et à cliquet.

14. Dispositif selon la revendication 12, **caractérisé en ce que** le chapeau de sûreté (8) présente, en son intérieur, un cylindre de protection (16) entourant l'aiguille pour injection (1).

15. Dispositif selon l'une quelconque des revendications 7 à 14, **caractérisé en ce que** le dispositif de pénétration de l'aiguille (17, 18) possède une face frontale (11"") terminale située à l'opposé du dispositif pour injection (12) et **en ce que** l'entretoise (21) se termine par une face frontale extérieure (21a) avec la face frontale (11"").
